# FASCICULE DE BREVET EUROPEEN

(11) **EP 4 330 648 B1**
(45) Date de publication et mention de la délivrance du brevet: **25.06.2025**
(21) Numéro de dépôt: 22724459.7
(22) Date de dépôt: 21.04.2022
(51) Int. Cl.: G01N 1/22, G01N 1/24, G01N 33/00, G01N 33/22, G01N 1/02

(54) **PROCEDE D'EXTRACTION ET DE DETECTION DE MOLECULES ODORANTES REELES D'INTERET ET DISPOSITIF D'EXTRACTION METTANT EN OEUVRE LEDIT PROCEDE**
VERFAHREN ZUR EXTRAKTION UND ERKENNUNG VON BESTIMMTEN RIECHENDEN MOLEKÜLEN UND EXTRAKTIONSVORRICHTUNG ZUR DURCHFÜHRUNG DES VERFAHRENS
METHOD FOR EXTRACTING AND DETECTING REAL ODOROUS MOLECULES OF INTEREST AND EXTRACTION DEVICE IMPLEMENTING SAID METHOD

(30) Priorité: 26.04.2021 FR 2104307
(43) Date de publication de la demande: 06.03.2024
(73) Titulaire: Biodesiv Sàrl, 2000 NEUCHATEL (CH)
(72) Inventeur: COURRIER, Quentin, 2000 NEUCHATEL (CH); HERIN, Grégory, 2000 NEUCHATEL (CH)
(74) Mandataire: Ipsilon Lyon
(86) Numéro de dépôt international: PCT/EP2022/060634
(87) Numéro de publication internationale: WO 2022/229009

(56) Documents cités:
- EP-B1- 3 621 431
- WO-A1-2018/206808
- DE-A1- 102015 002 161
- FR-A1- 2 956 211
- FR-A1- 3 088 429
- US-A1- 2008 250 878
- US-A1- 2010 045 459
- US-A1- 2012 316 677

## Description

### Domaine de l'invention

La présente invention se rapporte au domaine du contrôle et de la sûreté d'enceintes de toute taille par le biais de la collecte de molécules odorantes réelles d'intérêt. Ainsi, l'invention concerne un procédé et un dispositif pour la collecte, le stockage, le transport et l'analyse de molécules odorantes réelles d'intérêt contenues dans l'atmosphère d'une ou à proximité d'une enceinte à partir d'un échantillon de ladite atmosphère.

### Etat de la technique antérieure

La capacité à déceler des odeurs associées à des dangers ou des pathologies est connue et exploitée depuis très longtemps. Ainsi, les animaux de détection sont mis à contribution dans différents domaines tels que la santé, l'environnement, la détection et la répression voire même l'archéologie. Parmi les animaux de détection, le chien est le plus couramment mis à contribution puisqu'en moyenne, chaque chien inhale et expire environ cinq fois par seconde et capte ainsi différentes senteurs qui sont instantanément décodées par quelques 200 millions de cellules réceptrices présentes dans son nez. Par comparaison, l'Homme n'en possède que 5 millions en moyenne, ce qui rend le système olfactif du chien environ 40 fois plus sensible, et donc plus performant, que celui de l'Homme.

Parmi les applications les plus courantes de l'odorat du chien au service de l'Homme, on peut citer la recherche d'explosifs, de stupéfiants ou de restes humains, le pistage ou encore la détection de maladies.

Il est également possible de détecter des odeurs au moyen de différentes machines capables de séparer, identifier voire quantifier les molécules qui composent l'odeur. La technique la plus courante est la chromatographie par phase gazeuse couplée à un spectromètre de masse. Pour cela, il est nécessaire de collecter l'odeur afin de l'introduire dans l'appareil de mesure et ce, sur un support de petite taille compatible avec l'équipement. La sensibilité actuelle de ces techniques reste environ 1000 fois inférieure à celle de certains animaux, notamment les chiens. Aussi il apparaît nécessaire de pouvoir concentrer l'odeur lors de sa collecte pour pouvoir l'analyser.

**Concernant** en particulier le domaine de la détection et de la répression, toutes les cargaisons/marchandises de fret en circulation ne peuvent pas être contrôlées. Par exemple, dans les ports européens, moins de 5% des conteneurs sont contrôlés pour détecter une éventuelle présence actuelle ou passée de produits d'intérêt. Toutefois, ces contrôles peuvent s'avérer longs et coûteux, ce qui est à l'origine de leur faible fréquence. Au contraire, les États-Unis ont rendu obligatoire le contrôle de l'intégralité du fret aérien depuis début 2021.

Certains contrôles sont effectués par un scanner à rayons-X, mais la définition du scanner ne permet pas de détecter les produits d'intérêt de petite taille, disséminés dans la cargaison. A l'exception de quelques produits facilement reconnaissables, comme les cigarettes ou les armes, les scanners à rayons-X ne donnent pas d'indication précise sur la composition des éléments scannés, notamment sur leur caractère dangereux ou illicite. Les investissements dans ces outils sont également très élevés et nécessitent un raccordement électrique, limitant leur utilisation à certains terrains.

En parallèle, un autre type de contrôle consiste en des inspections visuelles/fouilles manuelles assistées ou non par des chiens de détection entrainés à reconnaître les odeurs des produits d'intérêt. Dans ce cas, les fouilles sont souvent partielles en ce qu'elles se limitent aux zones facilement accessibles par l'Homme ou le chien. Une inspection complète nécessite de déballer puis remballer la marchandise, la durée d'inspection est alors très importante tout comme le risque de blesser l'agent en charge du contrôle, le chien ou d'endommager la marchandise.

Les systèmes de contrôle par aspiration/filtration de microparticules apportent une solution à ces problématiques. Toutefois, ces systèmes restent très limités en termes de performance. En effet, ils ne fonctionnent que s'ils sont capables de recueillir suffisamment de microparticules dans l'air ambiant. Or, certains produits à contrôler ne produisent pas de microparticules ou sont emballés de sorte à empêcher leur dissémination. De plus, ces systèmes deviennent inopérants lorsque les cargaisons contiennent une grande quantité de particules dites « contaminantes », telles que des particules de poussière ou des matières pulvérulentes, qui peuvent boucher le filtre présent sur le dispositif d'aspiration/filtration. Par ailleurs, pour assurer l'aspiration d'un volume d'air important en un temps le plus court possible, le filtre doit présenter une perte de charge la plus faible possible. La perte de charge est directement liée à la porosité du filtre. Pour obtenir une faible perte de charge, il convient donc d'utiliser un filtre grossier (grande taille de mailles), ce qui retient peu ou pas les particules de petite taille et conduit à une mauvaise détection. Dans le cas particulier d'une cargaison contenant beaucoup de particules (ex. : farines, poussières, etc.) le filtre peut rapidement se colmater et devient alors inopérant. Au surplus, les filtres peuvent retenir des parasites et leurs œufs ou encore des bactéries et virus qui seraient présents dans la cargaison et qui exposeraient le chien de détection à un risque infectieux. Enfin, la dimension des filtres à particule n'est également pas compatible avec les appareils de séparation, détection voire quantification des molécules composant les odeurs.

Il ressort de ce qui précède qu'il est primordial de pouvoir effectuer des contrôles de cargaison le plus rapidement possible, indépendamment de la nature de la cargaison et avec un haut niveau de fiabilité, augmentant ainsi le nombre de saisies, tout en évitant les risques pour les agents et les chiens chargés du contrôle des marchandises, en limitant la dégradation des marchandises et en ayant la possibilité, le cas échéant, de détecter au moyen d'un équipement de séparation, identification voire quantification des molécules composant une odeur

En parallèle, des animaux, en particulier des chiens, sont couramment utilisés pour détecter la présence d'organismes nuisibles dans les bâtiments tels que les hôtels, les habitations privées ou encore les bureaux. Ces organismes sont, par exemple, les punaises de lit ou certaines moisissures difficilement repérables par l'Homme. Les animaux de détection, notamment les chiens, doivent parfois intervenir dans des lieux encombrés (caves, box de stockage, domicile d'une personne atteinte du syndrome de Diogène, etc.), connus pour être des réservoirs à colonies d'insectes ou de champignons.

Les solutions actuelles pour inspecter des lieux potentiellement contaminés par des organismes non désirés, consistent à faire sentir au chien tous les recoins de la pièce concernée. Si celle-ci est encombrées, cela oblige le chien à sentir la pièce de loin (perte d'efficacité de la détection), à déplacer les objets un par un par des opérateurs (risque de contamination de l'Homme, de blessure et/ou d'endommager des objets) ou encore à faire évoluer le chien sur les objets (risque de blessure pour le chien). Le dispositif de collecte par filtration n'est actuellement pas utilisé du fait de son caractère encombrant et la nécessité d'avoir une source de courant électrique proche (réseau électrique ou groupe électrogène).

Il ressort de ce qui précède, qu'il est primordial de pouvoir analyser ces bâtiments avec un équipement analytique ou un animal de détection, sans risque pour l'agent chargé du contrôle et l'animal de détection, en vue de leur apporter le traitement adéquat.

Concernant les maladies nosocomiales, il s'agit d'infections contractées dans un établissement de santé. On estime qu'en 2050, ces infections pourraient être à l'origine du décès de 10 millions de patients par an et induire un coût de prise en charge d'environ 100 000 milliards de dollars. Des procédures existent pour désinfecter les lieux sensibles. Toutefois, la désinfection n'est jamais efficace à 100% et la sur-utilisation de produits antiseptiques conduit au développement de résistances des bactéries et virus à l'action de ses produits antiseptiques. De plus, l'introduction d'appareils de mesure ou d'animaux de détection, comme un chien, dans une pièce aseptique élève le risque de re-contamination voire de sur-contamination de la pièce.

En conséquence, il existe un besoin de détecter la présence potentielle d'agents pathogènes dans ces pièces sans induire leur contamination.

Les documents US 2008/250878 A1 et FR 2 956 211 A décrivent des dispositifs de collecte sous la forme d'éléments plans, disposés perpendiculairement à la circulation de l'air aspiré et à travers lequel le flux d'air aspiré est forcé. La surface d'échange entre l'air aspiré et le dispositif de collecte est limitée et il est nécessaire d'utiliser un moyen d'aspiration puissant pour forcer l'air à travers ces dispositifs de collecte. En outre, dans une atmosphère chargée de poussières ou de particules en suspension, on rencontre fréquemment des problèmes de bouchage.

Le document FR 3 088 429 A1 décrit un dispositif de collecte sous la forme d'un cylindre plein ou d'un élément plan présentant les mêmes inconvénients que ci-dessus. Le caractère plein de ces éléments entraine des pertes de charge importantes rendant nécessaire de doter le dispositif d'un moyen d'aspiration puissant ainsi que d'un système de recirculation d'air pour que la quantité de molécules odorantes absorbées soit suffisante. Le système de recirculation présente un risque de pollution du sorbant par les odeurs/particules relarguées par les matériaux du système de recirculation eux-mêmes ou par des restes d'une précédente collecte. Le nettoyage est complexe, coûteux et sans garantie de résultat.

Il ressort des éléments qui précèdent qu'il existe un besoin évident de mettre en place un dispositif de détection qui soit efficace, fiable, rapide, qui s'adapte à différents domaines d'utilisation (contrôle et sureté, santé, environnement, etc.), et qui permette une détection par un animal ou un équipement analytique tout en réduisant voire annihilant les risques potentiels pour l'agent chargé du contrôle, l'animal de détection et/ou les marchandises/objets présents dans la zone de détection.

### Description de l'invention

Afin de résoudre ces problèmes, l'invention propose une solution simple d'utilisation, rapide, fiable et impliquant un faible investissement de départ pour sa mise en place. La solution proposée consiste en une collecte dynamique de molécules odorantes réelles qui sont caractéristiques du produit d'intérêt.

Au sens de l'invention, par « molécule odorante réelle » on désigne une molécule émise par une substance et qui peut être perçue par l'olfaction. Il s'agit de préférence d'une molécule organique, en général volatile, de faible masse moléculaire, en pratique inférieure à 350 g/mol, ce qui lui permet de se vaporiser à température ambiante pour qu'au moins une fraction se retrouve dans l'atmosphère gazeuse environnant le produit/la matière dont est issu cette molécule odorante. En outre, ces molécules peuvent aisément diffuser au travers de tissus ou de films plastiques.

Ainsi, selon un premier aspect, l'invention concerne un procédé dynamique de détection de molécules odorantes réelles d'intérêt contenues dans l'atmosphère ou à proximité directe de l'atmosphère présente dans une enceinte comprenant les étapes suivantes :
a) aspirer un échantillon de l'atmosphère de ladite enceinte au moyen d'un dispositif d'extraction ;
b) faire circuler ledit échantillon au travers et/ou sur un dispositif de collecte apte à adsorber puis absorber les molécules odorantes réelles d'intérêt, ledit dispositif se présentant sous la forme d'un tube creux ouvert à chacune de ses extrémités ; et
c) faire analyser ledit dispositif afin de déterminer si l'enceinte contient ou a contenu des produits d'intérêt.

Le procédé selon l'invention est un procédé dynamique d'extraction, collecte et détection de molécules odorantes réelles. En d'autres termes, il ne s'agit pas d'un dispositif de collecte statique qui reste en contact ou à proximité d'une source de molécules odorantes réelles afin de les collecter comme décrit dans le document WO 2018/206808.

Selon l'invention, par « à proximité directe de l'atmosphère présente dans une enceinte » on désigne une faible distance, avantageusement une zone située à moins de 2 mètres, voire moins d'1 mètre 50, moins d'1 mètre, moins de 50 centimètres ou encore moins de 30 centimètres.

Selon l'invention le dispositif se présente sous la forme d'un tube creux ouvert à chacune de ses extrémités de sorte que l'air contenant les molécules odorantes peut passer autour, mais aussi à l'intérieur du tube, ce qui augmente la surface d'absorption.

Une quantité suffisante de molécules odorantes peut ainsi être absorbée dès le premier passage de l'air et il n'est pas nécessaire de prévoir un système de recirculation. Le dispositif d'extraction selon l'invention aspire l'air de manière continue, sans qu'il soit nécessaire d'établir des cycles successifs d'aspiration, de recirculation, puis d'éjection. Le procédé et le dispositif selon l'invention sont donc plus simples et plus efficaces.

En outre, comme l'air peut facilement circuler à travers le tube creux, le débit d'air utilisé peut être plus important. Il y a moins de perte de charge et les risques d'obstruction sont éliminés. On peut donc se contenter d'un moyen d'aspiration plus simple, compact et peu coûteux, tel qu'un aspirateur domestique par exemple.

Selon un mode de réalisation particulier, l'aspiration de l'étape a) peut être réalisée en mode continu ou discontinu.

Selon un mode de réalisation particulier, la circulation de l'échantillon de l'étape b) est réalisée en continu, c'est-à-dire que l'échantillon est aspiré puis circule immédiatement et sans délai au travers du dispositif de collecte.

Selon un autre mode de réalisation, l'étape b) est réalisée en discontinu, c'est-à-dire que l'échantillon est aspiré puis stocké dans un réservoir. Il est ensuite soufflé au travers ou sur le dispositif de collecte.

Selon l'invention, l'analyse de l'étape c) peut être réalisée par un animal de détection et/ou une machine d'analyse. A titre d'exemple, l'animal de détection est un chien, un rongeur comme un rat ou encore des insectes comme des abeilles. Avantageusement l'animal de détection est un chien. L'animal, en particulier le chien, présente l'avantage d'identifier l'origine des molécules d'intérêt en une fraction de seconde, à la différence d'une machine d'analyse. En ce qui concerne les machines d'analyse, la technique la plus courante est la chromatographie par phase gazeuse couplée à un spectromètre de masse. Pour cela, il est nécessaire de collecter l'odeur afin de l'introduire dans l'appareil de mesure et ce, sur un support de petite taille compatible avec l'équipement.

Selon l'invention, le dispositif d'extraction et de collecte est mis en présence (directe ou à proximité) de l'atmosphère contenant ou ayant contenu une source de molécules odorantes réelles afin d'absorber lesdites molécules odorantes puis est définitivement écarté de la source de molécules odorantes avant d'être soumise à l'étape d'analyse (étape c)) du procédé de l'invention.

Au sens de l'invention, par « atmosphère », on désigne l'air, l'oxygène, l'hydrogène, l'azote, etc.

Selon un mode de réalisation particulier, l'échantillon d'atmosphère présent ou à proximité de l'enceinte selon l'invention représente au moins 2,5% du volume de ladite enceinte, avantageusement au moins 5%, 10%, 15%, 20% voire 25%

Selon un mode de réalisation particulier, le débit volumique d'aspiration du procédé de l'invention est déterminé en fonction du volume de l'échantillon. Selon l'invention, plus le débit volumique d'aspiration est élevé, plus la quantité de molécules odorantes réelles aspirées par le dispositif d'extraction/collecte de l'invention est importante. En d'autres termes, plus l'écoulement de fluide est important, plus le flux/l'écoulement de fluide passant au travers du dispositif de collecte apte à absorber les molécules odorantes réelles d'intérêt est important et donc, un volume de fluide plus important entre en contact avec ledit dispositif de collecte assurant qu'une grande quantité de molécules odorantes réelles soient absorbées par ledit dispositif.

Ainsi, le procédé de l'invention permet de faire passer un fluide contenant des molécules odorantes réelles d'intérêt avec un débit précis et spécifique sur le système de collecte afin qu'une quantité maximale de ces molécules odorantes soient absorbées.

Selon un mode de réalisation particulier, les molécules odorantes réelles d'intérêt selon l'invention sont issues d'une source de molécules odorantes choisies dans le groupe comprenant les stupéfiants, les explosifs, les armes, les munitions, les billets de banque, les moisissures, les organismes vivants tels que les insectes, les parasites, les bactéries, les champignons ou les virus ; des produits dits produits de contrebande tels que des drogues, des cigarettes, des animaux protégés ou encore des parties d'animaux protégés.

Selon un mode de réalisation particulier, l'enceinte selon l'invention est une cargaison ; avantageusement un conteneur, une remorque ou une palette, éventuellement réfrigérée ou congelée ; une pièce ou un bâtiment, l'intérieur ou l'extérieur d'un véhicule. A titre d'exemple, la cargaison selon l'invention peut être celle d'un bateau, d'un avion, d'un camion ou encore d'un train.

Selon un autre aspect, l'invention concerne un dispositif d'extraction pour la mise en œuvre du procédé tel que décrit précédemment, comprenant :
- un moyen d'aspiration de l'échantillon ;
- un dispositif de collecte apte à absorber les molécules odorantes réelles d'intérêt, ledit dispositif se présentant sous la forme d'un tube creux ouvert à chacune de ses extrémités ; et
- un moyen de fixation du dispositif de collecte au moyen d'aspiration.

Selon un mode de réalisation particulier, le moyen d'aspiration de l'échantillon est un aspirateur professionnel ou domestique ou une pompe présentant, un débit d'air avantageusement compris entre 0,1 et 10 m³/h, de préférence de l'ordre de 4 m³/h.

Selon l'invention, plus ce débit est élevé, plus la capacité de l'aspirateur à transporter l'air de la tête d'aspiration au réservoir est élevée.

Ce mode de réalisation particulier implique l'achat d'un aspirateur professionnel ou domestique dont le coût est largement inférieur à celui d'autres systèmes d'aspiration, notamment ceux utilisés conventionnellement. En outre, un aspirateur professionnel ou domestique est facilement disponible et est complètement compatible avec le dispositif de l'invention. L'invention permet également à l'utilisateur de travailler avec un aspirateur sur batterie, il dispose ainsi d'un outil léger, de petite taille et très maniable.

Selon l'invention, le dispositif de collecte apte à absorber les molécules odorantes réelles est un dispositif de collecte, d'absorption (ou imprégnation) et de relargage sur une longue période de ces molécules. Ce dispositif de collecte assure le stockage des molécules odorantes en vue d'une utilisation ultérieure, ce qui limite les risques pour l'agent chargé du contrôle et l'animal puisqu'ils ne sont jamais en contact direct avec la source de molécules odorantes, voire même avec l'atmosphère de l'enceinte.

En fonction de la dangerosité des molécules à détecter et des enceintes susceptibles de les contenir, le déplacement du dispositif d'extraction de l'invention est automatisé pour éviter tout risque pour l'agent et pour le chien. En d'autres termes et dans un mode de réalisation particulier, l'invention concerne également un robot motorisé qui comprend un dispositif d'extraction tel que précédemment décrit.

Le robot qui porte le dispositif d'extraction peut ainsi par exemple être autonome, téléguidé ou préprogrammé pour se déplacer jusqu'au lieu de l'extraction grâce à sa motorisation, sans que l'agent n'ait à s'approcher. Une fois sur place, le dispositif d'extraction est mis en fonctionnement soit directement par le robot, soit à distance par l'agent ou tout autre système, pour que l'échantillon d'atmosphère soit aspiré et les molécules odorantes collectées. Grâce à sa motorisation, le robot peut ensuite se déplacer jusqu'à l'endroit où le dispositif de collecte sera analysé ou jusqu'à un lieu éloigné de l'enceinte où il sera pris en charge par l'agent.

De tels robots motorisés, capables de se déplacer de manière autonome, téléguidé ou préprogrammé jusqu'à un lieu donné, sont connus de l'art antérieur et l'homme du métier pourra aisément en choisir un, compatible avec le dispositif d'extraction et le procédé selon l'invention, sans qu'il soit nécessaire de le décrire plus en détail.

L'invention permet donc de collecter rapidement les molécules odorantes présentes dans un grand volume et de les concentrer dans un dispositif de collecte apte à absorber les molécules odorantes réelles d'intérêt. Ce dispositif apte à absorber les molécules odorantes possède la capacité de les stocker en grande quantité, puis de les restituer de manière contrôlée pendant une longue période.

Selon un mode de réalisation particulier, le dispositif de collecte apte à absorber les molécules odorantes réelles selon l'invention présente une neutralité olfactive.

Selon un mode de réalisation particulier, le dispositif de collecte apte à absorber les molécules odorantes réelles selon l'invention comprend, avantageusement est constitué, d'un polymère ou d'un mélange de polymères permettant une absorption des molécules odorantes d'intérêt dans le maillage de son réseau polymérique. Ainsi, les molécules odorantes réelles ne sont pas simplement adsorbées mais absorbées dans le dispositif. Une grande quantité de molécules odorantes peut ainsi être stockée puis restituée lors de l'utilisation du dispositif, notamment pour l'étape c) du procédé selon l'invention. Avantageusement, la restitution des molécules odorantes selon l'invention ne nécessite pas d'étape de désorption dudit dispositif autre qu'une désorption naturelle lorsque la détection est opérée par un animal tel qu'un chien par exemple.

La structure du ou des polymères utilisé(s) dans l'invention est différente de la structure des molécules odorantes d'intérêt à collecter, notamment dans le but de ne pas induire de biais dans l'étape d'analyse du procédé de l'invention (étape c)). En outre, ladite structure du ou des polymères est chimiquement compatible avec la molécule d'intérêt pour qu'il puisse y avoir absorption. La molécule odorante d'intérêt est donc au moins partiellement soluble dans le(s) polymère(s) constituant le dispositif pour pouvoir y être absorbée et stockée de manière passive.

Selon un mode de réalisation particulier, le dispositif de collecte apte à absorber les molécules odorantes réelles selon l'invention n'est pas pré-imprégné par une odeur naturelle ou synthétique (pseudo-odeur) et il ne contient pas de particules de matière réelle de la source de molécules odorantes.

Selon un mode de réalisation particulier, le dispositif de collecte apte à absorber les molécules odorantes réelles selon l'invention comprend, avantageusement est constitué, d'un polymère pur ou d'un mélange de polymères, des additifs peuvent éventuellement y être ajoutés. Ce matériau présente une haute capacité d'absorption de molécules odorantes de différentes natures, que celles-ci soient polaires ou apolaires, et notamment des COV (composés organiques volatils). Il présente également la capacité de relarguer les molécules odorantes qu'il contient de manière contrôlée en débit et en durée. Enfin il est facile à mettre en œuvre par les procédés classiques de mise en œuvre des polymères, à savoir et de manière non limitative : extrusion, filage, injection, pressage, casting, rotomoulage, extrusion soufflage, étirage soufflage, extrusion gonflage, thermoformage, moulage par compression, etc.

Selon un mode de réalisation particulier, dans le cas où le dispositif de collecte apte à absorber les molécules odorantes réelles selon l'invention comprend, avantageusement est constitué de, un mélange de polymères, si ces derniers ne sont pas miscibles alors ils sont mélangés dans des proportions relatives permettant l'obtention d'un réseau polymérique co-continu. Dans ce contexte les polymères du mélange sont présents à la surface pour être au contact des molécules odorantes à capter lors de l'absorption. Chaque type de polymère formant un réseau continu, les molécules odorantes peuvent donc par diffusion être stockées dans tout le volume de la matière du dispositif selon l'invention.

Selon un mode de réalisation particulier, le ou les polymère(s) utilisé(s) pour réaliser un dispositif selon l'invention sont sélectionnés dans le groupe constitué par des polymères naturels, artificiels ou synthétiques, avantageusement des polymères artificiels. Alternativement, il peut s'agir d'un mélange entre au moins deux polymères de différentes natures (ex. polymère naturel avec polymère synthétique). Parmi les polymères naturels aptes à être mis en œuvre dans l'invention, on peut citer les polysaccharides, les glycosamininoglycanes, la lignine, les acides nucléiques, les scléroprotéines, le caoutchouc naturel et les polyhydroxyalkanoates. Parmi les polymères artificiels aptes à être mis en œuvre dans l'invention, on peut citer l'acétate de cellulose et la nitrocellulose. Parmi les polymères synthétiques aptes à être mis en œuvre dans l'invention, on peut citer les polyoléfines , les polymères vinyliques, les polymères styréniques, les polyesters, les polyamides, les polyuréthanes, les polycarbonates, les polymères acryliques, les polymères aminoplastes et phénoplastes, les polyacétals, les polymères silicones, les polyimides, les polymères halogénés, les polydiméthylsiloxane, les époxydes, les polymères thermostables, les élastomères et les polymères électroactifs.

Selon la présente invention le dispositif de collecte et de restitution de molécules odorantes réelles d'intérêt comprend, est constitué de, un polymère ou d'un mélange de polymères, présentant avantageusement une neutralité olfactive, sélectionné dans le groupe constitué par le polyéther-bloc-amide (PEBA), le polypropylène (PP), les polyoléfines, un mélange de polyéther-bloc-amide avec un ou plusieurs polymères de type silicone (PDMS), et des polymères aromatiques.

Selon un mode de réalisation particulier, le polymère selon l'invention est sélectionné dans le groupe constitué par le polyéther-bloc- amide (ou PEBA), un mélange de polyéther-bloc-amide avec un ou plusieurs polymères de type polyoléfine (polyéthylène ou polypropylène), un mélange de polyéther-bloc-amide avec un ou plusieurs polymères de type silicone (PDMS) , un mélange de polyéther-bloc-amide avec un ou plusieurs polymères aromatiques, par exemple et de manière non limitative un polystyrène ou un mélange de polyéther-bloc-amide avec un ou plusieurs polymères. Plus généralement le polymère ajouté au PEBA est choisi parmi ceux permettant d'absorber puis de relarguer une ou plusieurs molécules odorantes spécifiques à une application.

Selon un mode de réalisation particulier, le polymère est le PEBA seul ou en combinaison avec un autre polymère.

Selon un mode de réalisation particulier, le dispositif de collecte qui se présente sous la forme d'un tube creux ouvert à chacune de ses extrémités, est avantageusement en PEBA ou réalisé dans l'un des polymères ou mélange de polymères précités.

Selon un mode de réalisation particulier, le moyen de fixation du moyen de collecte au moyen d'aspiration selon l'invention se présente sous la forme d'un manchon, de préférence de forme générale tubulaire, comprenant :
- une paroi ;
- une première extrémité débouchante apte à aspirer l'échantillon d'atmosphère ;
- une seconde extrémité, de préférence opposée à la première extrémité apte à être connectée au moyen d'aspiration ; et
- un moyen de retenue du moyen de collecte.

Selon un mode de réalisation particulier, l'extrémité débouchante du manchon présente au moins un moyen de connexion à un prolongateur, ledit prolongateur étant par exemple, du type tube souple de diamètre variable en fonction des applications et qui puisse être facilement inséré dans une cargaison de fret ou dans une pièce, par exemple par l'interstice d'une porte, au travers du trou d'une serrure, etc.

A titre d'exemple, le au moins un moyen de connexion se présente sous la forme d'un taraudage ou d'un filetage destiné à coopérer avec un moyen correspondant et prévu sur le prolongateur.

Pour permettre la connexion de l'autre extrémité du manchon au moyen d'aspiration, celle-ci présente également au moins un moyen de connexion, de préférence clipsable. Il peut s'agir par exemple d'éléments mâle/femelle destinés à coopérer avec des éléments correspondants du moyen d'aspiration.

Selon l'invention, le manchon est muni d'un moyen de retenue du dispositif de collecte sous la forme par exemple d'un logement destiné à recevoir ledit dispositif de collecte. Avantageusement, le logement est de forme générale tubulaire tronconique pour permettre de fixer efficacement le dispositif de collecte, en pratique le tube, par insertion.

Selon un mode de réalisation particulier, la paroi du logement comporte au moins un évidement, de sorte à permettre à l'atmosphère aspiré d'être, notamment, en contact direct avec la surface extérieure du dispositif de collecte maintenu dans le logement.

Selon l'invention, le dispositif de collecte est aisément retiré du logement et donc du dispositif d'extraction pour être ensuite analysé (ex. : chien de détection, GC/MS, etc.) de sorte à confirmer la présence ou non, actuelle ou passée, de produits d'intérêt dans l'enceinte soumise à une inspection.

En pratique, le dispositif de collecte doit être manipulé pour être introduit et extrait du logement. Cette manipulation s'effectue manuellement, en particulier par un agent portant des gants à usage unique ou sans manipulation directe, c'est-à-dire à l'aide d'un outil, par exemple une pince.

En pratique, le moyen de retenue du dispositif de collecte est maintenu, avantageusement au centre du manchon, par le biais d'au moins un moyen de liaison à la paroi dudit manchon, par exemple sous la forme d'une ou plusieurs ailettes.

Selon un mode de réalisation particulier, le manchon contient 4 ailettes placée à 90° les unes par rapport aux autres.

Selon un mode de réalisation particulier, le dispositif d'extraction selon l'invention est transportable. A titre d'exemple, ledit dispositif peut être transporté par un agent chargé d'un contrôle, un animal, voire même un engin motorisé tel qu'un robot.

Avantageusement, le dispositif d'extraction selon l'invention peut être activé/désactivé manuellement et/ou à distance par télécommande.

Selon un mode de réalisation particulier, le dispositif d'extraction est en acier inoxydable et/ou en matière plastique.

Il ressort de ce qui précède que le dispositif d'extraction selon l'invention a pour avantages de:
- présenter une forme assurant une perte de charge minimale afin de garantir un haut débit d'aspiration ;
- être le plus léger possible (présence d'évidemment et matériaux de fabrication légers) et donc facilement transportable et maniable ;
- se composer de matériaux durables et standards, notamment dans l'industrie du médical (par exemple l'acier inoxydable 316L).

En outre et contrairement au dispositif de collecte par filtration de l'art antérieur qui vise à collecter des particules en suspension directement émises par le produit d'intérêt, l'invention présente les avantages suivant de :
- collecter les molécules odorantes qui forment la signature olfactive du produit d'intérêt ;
- pouvoir détecter des éléments d'intérêt (produits, animaux) qui n'émettent pas de particules dans l'air (via un processus naturel ou en raison de leur emballage) ; et
- pouvoir détecter la présence passée d'éléments d'intérêt.

Selon un autre aspect, le dispositif comprend un moyen de fixation d'un dispositif de collecte apte à absorber les molécules odorantes réelles d'intérêt destiné à être connecté au moyen d'aspiration du dispositif décrit précédemment. Il se caractérise en ce qu'il comprend un manchon comprenant :
- une paroi ;
- une première extrémité débouchante apte à aspirer l'échantillon d'atmosphère ;
- une seconde extrémité apte à être connectée au moyen d'aspiration ; et
- un moyen de retenue du dispositif de collecte.

Selon un autre aspect, l'invention concerne l'utilisation du dispositif d'extraction tel que décrit précédemment pour contrôler la légalité et/ou assurer la sûreté et/ou assurer la sécurité d'une cargaison, d'une pièce, d'un bâtiment ou encore d'un véhicule.

Selon un mode de réalisation particulier, l'utilisation du dispositif selon l'invention est réalisée dans au moins un des domaines suivants : contrôle douanier, fouille de véhicules, perquisitions dans le cadre d'enquêtes de police/gendarmerie, contrôle de la présence de produits d'intérêt dans des bâtiments, détection d'organismes indésirables (insectes, par exemple punaises ou micro-organismes) chez les particuliers, les professionnels recevant du public (hôtellerie) ou encore dans des établissements de santé (hôpitaux etc...).

Ainsi, la présente invention :
- permet d'effectuer des détections (ou contrôles) plus rapides grâce à un débit d'air plus important puisque le dispositif d'extraction de l'invention assure moins de perte de charge du fait de l'absence de filtre (diminution du coût/contrôle) ;
- est plus fiable du fait d'une technologie basée sur l'absorption de molécules odorantes qui sont plus faciles à collecter que des particules et plus représentatives de l'odeur réelle du produit d'intérêt (augmentation des saisies et donc diminution du manque à gagner pour les Etats) ;
- est universelle puisque tous les types de cargaison peuvent être contrôlés ou encore bâtiments ou véhicules ;
- garantie une meilleure sécurité pour les agents de contrôle, les animaux de détection ou encore la marchandise ;
- est mise en œuvre avec un investissement initial faible (car le dispositif s'adapte sur des systèmes d'aspiration du commerce) ;
- utilise des ressources déjà disponibles (douaniers, chien de détection) ;
- est plus discret en ce qu'il n'est pas toujours nécessaire de briser les scellés pour effectuer un contrôle, le tube d'aspiration pouvant se glisser, par exemple, sous la bâche d'un camion ; et
- est efficace puisque le dispositif de l'invention est maniable, de petite taille, de faible poids, donne la possibilité de travailler sur batterie donc dans des lieux sans raccordement électrique.

### Brève description des figures

La manière de réaliser l'invention, ainsi que les avantages qui en découlent, ressortiront bien de la description des modes de réalisation qui suivent, à l'appui des figures annexées dans lesquelles :

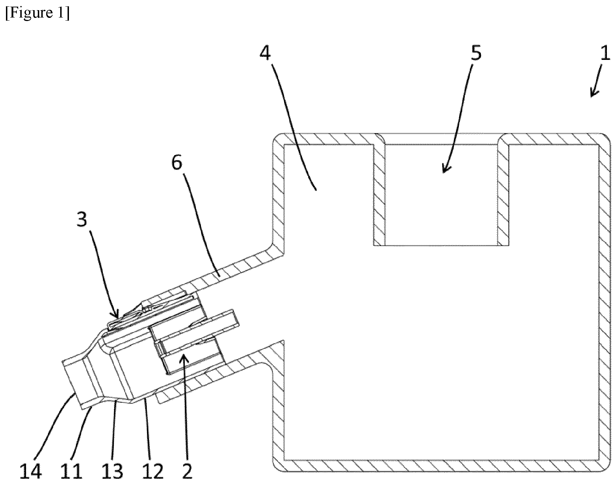

La figure 1 est une vue schématique en coupe du dispositif d'extraction de l'invention.
La figure 2 est une vue schématique vue de dessus du manchon du dispositif d'extraction.
La figure 3 est une coupe transversale de la figure 2 selon le plan AA.
La figure 4 est une vue en perspective du manchon en l'absence du dispositif de collecte.
La figure 5 est une vue schématique du dispositif de collecte de l'invention.

### Description des modes de réalisation

Le dispositif de l'invention comprend essentiellement trois éléments que sont un moyen d'aspiration (1), un dispositif de collecte (2) apte à absorber les molécules odorantes et un moyen de fixation (3) du dispositif de collecte (2) au moyen d'aspiration (1).

Le moyen d'aspiration (1) comprend essentiellement une cuve (4) de réception des poussières, un moteur (5) et un adaptateur (6) au moyen de fixation (3).

En pratique, le moyen d'aspiration est un aspirateur domestique, par exemple un aspirateur de la marque DYSON^{®}, sur lequel vient se connecter le moyen de fixation (3) en lieu et place des modules standards que l'on trouve dans le commerce.

### On peut utiliser un aspirateur avec un débit d'air d'environ 4 m³/h

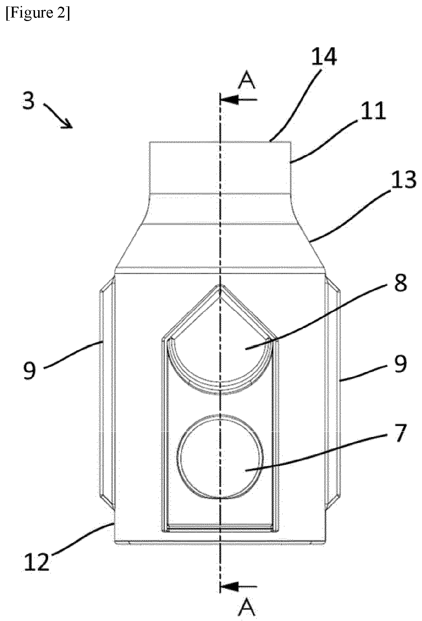

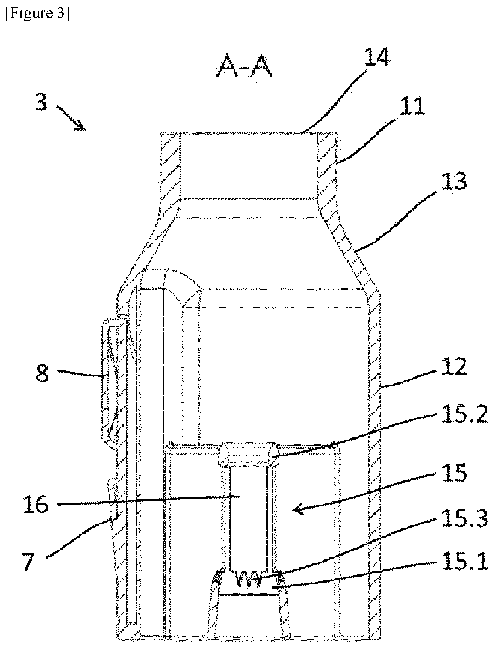

L'adaptateur (6) est destiné à être connecté au moyen de fixation (3) du dispositif de collecte (2) notamment par clipsage. Des moyens de clipsage sont plus particulièrement représentés sur les figures 2 à 4. Il s'agit dans ce mode de réalisation de deux boutons (7, 8) destinés à coopérer avec des ouvertures correspondantes agencées sur l'adaptateur (6) de l'aspirateur.

Les deux boutons (7,8) sont, toujours dans ce mode de réalisation particulier, doublés de nervures (9) destinées à coopérer avec des gorges non représentées correspondantes du manchon (6) permettant ainsi d'éviter tout jeu entre les deux éléments.

Le dispositif de collecte (2) est plus particulièrement représenté sur la figure (5). Il se présente sous la forme d'un tube (10) creux dont ses deux extrémités (10.1, 10.2) sont ouvertes de manière à laisser passer l'échantillon d'atmosphère aspiré. Dans ce mode de réalisation, le tube est fabriqué en PEBA mais peut être fabriqué dans toutes les matières telles que précédemment mentionné.

Le dispositif d'extraction selon l'invention comprend un troisième élément qu'est le moyen de fixation (3) du dispositif de collecte (2).

### Ce moyen de fixation est représenté sur les figures 2 à 4.

Il se présente sous la forme d'un manchon de forme générale tubulaire, présentant trois sections distinctes dans le sens de l'extraction de l'échantillon d'atmosphère respectivement une première section tubulaire (11), une seconde section tubulaire (12) de diamètre supérieur à celui de la première section (11), les deux sections (11, 12) étant reliées par une section conique (13). Cette forme générale du manchon permet d'optimiser la dynamique d'extraction de l'échantillon d'atmosphère et l'écoulement du fluide dans ledit manchon au travers du tube (10) creux comme au niveau de sa surface extérieure.

L'extrémité débouchante (14) du manchon peut être munie, dans certains cas, de moyens de connexion à un prolongateur non illustré. Le prolongateur se présente, en pratique, sous la forme d'un tube souple et destiné à être connecté à la paroi de la cargaison, telle que par exemple la paroi d'un container.

Le manchon (3) comporte en outre un moyen de retenue (15) du dispositif de collecte (2) se présentant lui-même sous forme générale tubulaire tronconique de manière à recevoir le dispositif de collecte par insertion. Le moyen de retenue est positionné au centre du manchon dans le sens longitudinal et présente ainsi 2 bagues respectivement une première bague (15.1) d'un diamètre légèrement supérieur à celui du tube collecteur et une seconde bague (15.2) de diamètre sensiblement égale à celui du tube collecteur. La première bague (15.1) est en outre munie sur sa périphérie en regard de la seconde bague (15.2) de dents (15.3) orientées en direction de la seconde bague. Ces dents permettent ainsi de maintenir en position le tube collecteur dans son logement.

La paroi du logement (15) présente en outre des évidements sous la forme de fenêtres (16) permettant à la surface du tube d'être au contact direct de l'échantillon d'atmosphère entrant par aspiration. En d'autres termes, la majeure partie des surfaces interne et externe du tube est en contact avec l'échantillon d'atmosphère extrait.

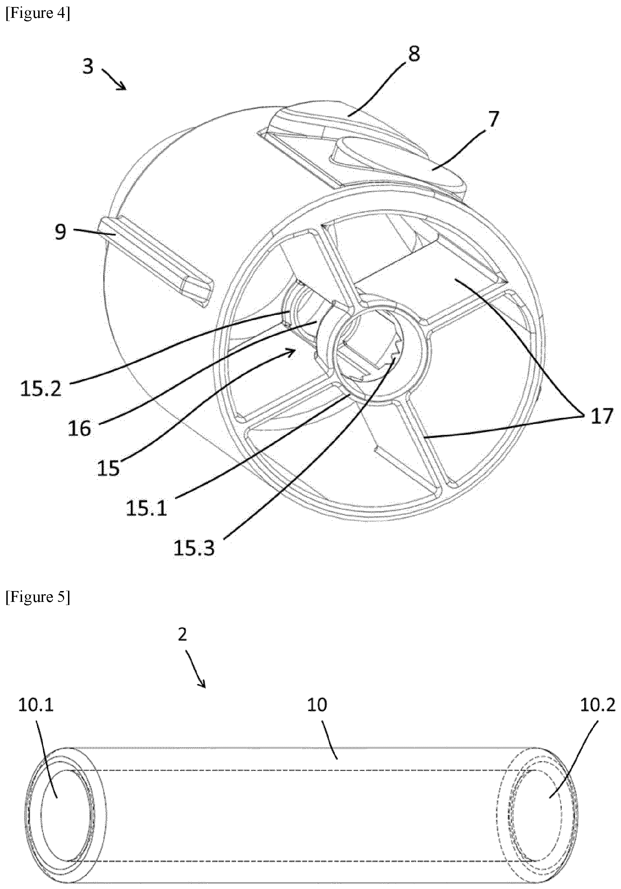

Comme le montre la figure 4, le logement (15) est relié à la paroi du manchon (3) par le biais d'ailettes (17) qui sont au nombre de 4 dans le mode de réalisation tel que représenté. Les ailettes sont réparties de manière symétrique tout autour du manchon.

En pratique, et comme expliqué précédemment, le tube collecteur est reçu dans un emballage imperméable aux odeurs. Il suffit alors à l'opérateur, après ouverture du blister, d'insérer le tube collecteur dans le logement (15) prévu à cet effet, par insertion, éventuellement à l'aide d'un outil comme une pince. Le manchon est ensuite connecté au dispositif d'aspiration (1) par le biais de l'adaptateur (6). Il suffit alors à l'opérateur d'activer l'aspirateur et d'aspirer un échantillon d'atmosphère à tester à proximité du container ou éventuellement directement dans le container, notamment par le biais d'un prolongateur.

En pratique, le volume de l'échantillon est déterminé en fonction de la nature des produits susceptibles d'être identifiés et/ou du volume de l'enceinte dans laquelle lesdits produits sont potentiellement présents. Ce volume peut aller de 2,5% à 10 % par exemple.

L'opération d'aspiration est très rapide de l'ordre de quelques minutes. Une fois l'aspiration effectuée, il suffit à l'opérateur de saisir le tube du moyen de retenue (15) et le présenter, immédiatement ou non, à un chien de détection. Le PEBA constitutif du tube permet en effet de fixer les odeurs sur une longue durée.

Il suffira alors au chien de quelques secondes pour identifier l'odeur et donc la relier à une matière ou un produit spécifique.

L'invention et les avantages qui en découlent ressortent bien de la description qui précède : on note notamment la facilité d'utilisation du dispositif de l'invention qui, combiné au dispositif de collecte, permet d'identifier la majeure partie des substances illicites et en particulier dans le cadre du contrôle de fret.

## Revendications

1. Procédé dynamique de détection de molécules odorantes réelles d'intérêt contenues dans l'atmosphère ou à proximité directe de l'atmosphère présente dans une enceinte comprenant les étapes suivantes :
a) Aspirer un échantillon de l'atmosphère présent ou à proximité de ladite enceinte au moyen d'un dispositif d'extraction ;
b) Faire circuler/passer ledit échantillon au travers et/ou sur un dispositif de collecte apte à absorber les molécules odorantes réelles d'intérêt, ledit dispositif se présentant sous la forme d'un tube creux ouvert à chacune de ses extrémités ; et
c) Faire analyser ledit dispositif par un animal de détection et/ou une machine d'analyse.

2. Procédé selon la revendication 1, **caractérisé en ce que** les molécules odorantes réelles d'intérêt sont issues d'une source de molécules odorantes sélectionnées dans le groupe constitué par les stupéfiants, les explosifs, les armes, les munitions, les billets de banque, les moisissures, les organismes vivants tels que les insectes, les parasites, les bactéries, les champignons ou les virus et les produits dits produits de contrebande tels que des drogues, des cigarettes, des animaux protégés ou encore des parties d'animaux protégés.

3. Procédé selon l'une quelconque des revendications précédentes, **caractérisé en ce que** l'enceinte est une cargaison ; avantageusement un conteneur, une remorque ou une palette, éventuellement réfrigérée ou congelée ; une pièce, un bâtiment ou un véhicule.

4. Dispositif d'extraction pour la mise en œuvre du procédé selon l'une des revendications 1 à 3, comprenant :
- un moyen d'aspiration (1) de l'échantillon ;
- un dispositif de collecte (2) apte à absorber les molécules odorantes réelles d'intérêt de l'échantillon, le dispositif se présentant sous la forme d'un tube creux (10) ouvert à chacune de ses extrémités (10.1, 10.2) ; et
- un moyen de fixation (3) du dispositif de collecte (2) au moyen d'aspiration, **caractérisé en ce que** le moyen de fixation est arrangé de façon à ce que l'air contenant les molécules odorantes de l'échantillon peut passer autour, mais aussi à l'intérieur du tube, ce qui augmente la surface d'absorption.

5. Dispositif d'extraction selon la revendication 4 , **caractérisé en ce que** le dispositif de collecte (2) est constitué d'un polymère sélectionné dans le groupe constitué par le polyéther-bloc- amide (ou PEBA), un mélange de polyéther-bloc-amide avec un ou plusieurs polymères de type polyoléfine (polyéthylène ou polypropylène), un mélange de polyéther-bloc-amide avec un ou plusieurs polymères de type silicone (PDMS), un mélange de polyéther-bloc-amide avec un ou plusieurs polymères aromatiques.

6. Dispositif d'extraction selon l'une des revendications 4 ou 5, **caractérisé en ce que** le moyen de fixation (3) se présente sous la forme d'un manchon comprenant :
- une paroi ;
- une première extrémité débouchante (14) apte à aspirer l'échantillon d'atmosphère ;
- une seconde extrémité apte à être connectée au moyen d'aspiration (1) ; et
- un moyen de retenue (15) du dispositif de collecte.

7. Dispositif d'extraction selon la revendication 6, **caractérisé en ce que** la première extrémité présente des moyens de connexion à un prolongateur, du type tube souple.

8. Dispositif d'extraction selon la revendication 6, **caractérisé en ce que** la seconde extrémité présente au moins un moyen de connexion (7,8) clipsable, du type éléments mâle/femelle destinés à coopérer avec au moins un élément correspondant du moyen d'aspiration.

9. Dispositif d'extraction selon la revendication 6, **caractérisé en ce que** le moyen de retenue (15) se présente sous la forme d'un logement de forme générale tubulaire tronconique apte à fixer le dispositif de collecte (2) par insertion.

10. Dispositif d'extraction selon la revendication 9, **caractérisé en ce que** la paroi du logement comporte au moins un évidement (16).

11. Dispositif d'extraction selon l'une des revendications 6 à 10, **caractérisé en ce que** le moyen de retenue est maintenu au centre du manchon par le biais de moyens de liaison à la paroi dudit manchon, par exemple sous la forme d'ailettes (17).

12. Dispositif d'extraction selon l'une des revendications 4 à 11, **caractérisé en ce qu'**il est transportable.

13. Robot motorisé **caractérisé en ce qu'**il comprend un dispositif d'extraction selon l'une des revendications 4 à 12.

## Patentansprüche

1. Dynamisches Verfahren zur Erkennung von realen, relevanten Geruchsstoffmolekülen, enthalten in der Atmosphäre oder in unmittelbarer Nähe der Atmosphäre in einem Behälter, das die folgenden Schritte umfasst:
a) Ansaugen einer Probe aus der im genannten Behälter oder in seiner Nähe vorhandenen Atmosphäre mittels einer Extraktionsvorrichtung;
b) Diese Probe auf einer Sammelvorrichtung zirkulieren lassen und/ oder darüberführen, die diese realen, relevanten Geruchsstoffmoleküle absorbieren kann, wobei diese Vorrichtung die Form eines hohlen, auf beiden Seiten offenen Röhrchens hat; und
c) Analyse dieser Vorrichtung durch tierische oder maschinelle Geruchsanalyse.

2. Verfahren nach Anspruch 1, **dadurch gekennzeichnet, dass** die realen, relevanten Geruchsstoffmoleküle aus einer Quelle von Geruchsstoffmolekülen stammen, die zu einer Gruppe mit Rauschgiften, Explosivstoffen, Waffen, Munition, Banknoten, Schimmelpilzen, lebenden Organismen wie Insekten, Parasiten, Bakterien, Pilzen oder Viren gehören sowie sogenannter Schmuggelware, wie Drogen, Zigaretten, geschützten Tierarten oder auch Teilen von geschützten Tierarten.

3. Verfahren nach irgendeinem der vorstehenden Ansprüche, **dadurch gekennzeichnet, dass** es sich bei dem Behälter um eine Ladung; vorteilhafterweise einen Container, einen Anhänger oder eine Palette, eventuell gekühlt oder tiefgekühlt; einen Raum, ein Gebäude oder ein Fahrzeug handelt.

4. Extraktionsvorrichtung zum Einsatz des Verfahrens nach einem der Ansprüche 1 bis 3, mit:
- einem Ansaugelement (1) für die Probe;
- einer Sammelvorrichtung (2), die diese realen, relevanten Geruchsstoffmoleküle der Probe absorbieren kann.
die Vorrichtung hat dabei die Form eines hohlen, auf beiden Seiten (10.1, 10.2) offenen Röhrchens (10); und
- ein Element zur Befestigung (3) der Sammelvorrichtung (2) am Ansaugelement. **dadurch gekennzeichnet, dass** das Befestigungselement so angeordnet ist, dass die Luft, die die Geruchsstoffmoleküle der Probe enthält, darum herumstreichen kann, aber auch durch das Innere des Röhrchens, was die Absorptionsfläche vergrößert.

5. Extraktionsvorrichtung nach Anspruch 4, **dadurch gekennzeichnet, dass** die Sammelvorrichtung (2) aus einem Polymer besteht, das zur Gruppe bestehend aus Polyether-Blockamid (oder PEBA), einer Mischung aus Polyether-Blockamid mit einem oder mehreren Polymeren vom Typ Polyolefin (Polyethylen oder Polypropylen), einer Mischung aus Polyether-Blockamid mit einem oder mehreren Polymeren vom Typ Silikon (PDMS) oder einer Mischung aus Polyether-Blockamid mit einem oder mehreren aromatischen Polymeren, gehört.

6. Extraktionsvorrichtung nach einem der Ansprüche 4 oder 5, **dadurch gekennzeichnet, dass** das Befestigungselement (3) die Form einer Hülse hat, umfassend:
- eine Wand;
- ein erstes Ende (14), das in der Lage ist, die Probe aus der Atmosphäre anzusaugen;
- ein zweites Ende, das mit der dem Ansaugelement (1) verbunden werden kann; und
- ein Halteelement (15) für die Sammelvorrichtung.

7. Extraktionsvorrichtung nach Anspruch 6, **dadurch gekennzeichnet, dass** das erste Ende Verbindungselemente zur Verbindung mit einer Verlängerung, vom Typ Schlauch aufweist.

8. Extraktionsvorrichtung nach Anspruch 6, **dadurch gekennzeichnet, dass** das zweite Ende mindestens ein aufsteckbares Verbindungselement (7,8) aufweist, vom Typ auf-/ einsteckbare Elemente, das mit mindestens einem Element zusammenarbeiten kann, das der Ansaugvorrichtung entspricht.

9. Extraktionsvorrichtung nach Anspruch 6, **dadurch gekennzeichnet, dass** das Halteelement (15) die allgemeine Form einer rohrförmigen, konischen Unterbringung hat, in der die Sammelvorrichtung (2) durch Einrasten befestigt werden kann.

10. Extraktionsvorrichtung nach Anspruch 9, **dadurch gekennzeichnet, dass** die Wand der Unterbringung mindestens eine Aussparung (16) enthält.

11. Extraktionsvorrichtung nach einem der Ansprüche 6 bis 10, **dadurch gekennzeichnet, dass** das Halteelement in der Mitte der Hülse über Verbindungselemente zur Wand dieser Hülse gehalten wird, zum Beispiel in Form von Rippen (17).

12. Extraktionsvorrichtung nach einem der Ansprüche 4 bis 11, **dadurch gekennzeichnet, dass** sie transportierbar ist.

13. Motorgetriebener Roboter, **dadurch gekennzeichnet, dass** er eine Extraktionsvorrichtung nach einem der Ansprüche 4 bis 12 enthält.

## Claims

1. A dynamic method for detecting actual odorous molecules of interest contained in the atmosphere or in direct proximity to the atmosphere present in an enclosure comprising the following steps:
a) Absorbing a sample of the atmosphere present in or proximate to said enclosure by means of an extraction device;
b) Making said sample circulate/pass through and/or over a collection device capable of absorbing the actual odorous molecules of interest, said device being in the form of a hollow tube open at each of its ends; and
c) Having said device analyzed by a detection animal and/or an analysis machine.

2. The method according to claim 1, **characterized in that** the actual odorous molecules of interest come from a source of odorous molecules selected from the group including narcotics, explosives, weapons, ammunition, bank notes, mold, living organisms such as insects, parasites, bacteria, fungi or viruses and so-called contraband products such as drugs, cigarettes, protected animals or parts of protected animals.

3. The method according to any one of the preceding claims, **characterized in that** the enclosure is cargo; advantageously a container, a trailer or a pallet, possibly refrigerated or frozen; a room, a building or a vehicle.

4. An extraction device for implementing the method according to any one of clains 1 to 3, comprising:
a suction means (1) for collecting the sample;
a collection device (2) capable of absorbing the actual odorous molecules of interest of said sample, the device being in the form of a hollow tube (10) open at each of its ends (10.1, 10.2); and
a means for fastening the collection device to the suction means, **characterized in that** the fastening means is arranged so that the air containing the odorant molecules of the sample can pass around, but also inside the tube, thus increasing the absorption surface.

5. The extraction device according to claim 4, **characterized in that** the collection device (2) consists of a polymer selected from the group consisting of polyether-block-amide (or PEBA), a mixture of polyether-block-amide with one or more polyolefin-type polymer(s) (polyethylene or polypropylene), a mixture of polyether-block-amide with one or more silicone-type polymer(s) (PDMS), a mixture of polyether-block-amide with one or more aromatic polymer(s).

6. The extraction device according to claim 4 or 5, **characterized in that** the fastening means (3) is in the form of a sleeve comprising:
- a wall;
- a first open end (14) capable of sucking in the atmosphere sample;
- a second end capable of being connected to the suction means (1); and
- a retaining means (15) of the collection device.

7. The extraction device according to claim 6, **characterized in that** the first end has means for connection to an extension, of a flexible tube type.

8. The extraction device according to claim 6, **characterized in that** the second end has at least one clip-on connection means (7, 8), of a male/female element type intended to cooperate with at least one corresponding element of the suction means.

9. The extraction device according to claim 6, **characterized in that** the retaining means (15) is in the form of a generally frustoconical tubular housing capable of fastening the collection device (2) by insertion.

10. The extraction device according to claim 9, **characterized in that** housing has a wall that includes at least one recess (16).

11. The extraction device according to any one of claims 6 to 10, **characterized in that** the retaining means is held at the center of the sleeve by means of connecting means to the wall of said sleeve, for example in the form of fins (17).

12. The extraction device according to any one of claims 4 to 11, **characterized in that** it is transportable.

13. A motorized robot **characterized in that** it comprises an extraction device according to any one of claims 4 to 12.
